(19) Europäisches Patentamt
European Patent Office
Office européen des brevets

(11) Publication number: **0 455 037 A2**

# (12) EUROPEAN PATENT APPLICATION

(21) Application number: **91105986.3**

(22) Date of filing: **15.04.91**

(51) Int. Cl.[5]: **C12Q 1/68**, C07H 21/04, C12P 19/34, //C07K13/00, C12N15/53,C12P21/02

(30) Priority: **16.04.90 JP 99622/90**

(43) Date of publication of application: **06.11.91 Bulletin 91/45**

(84) Designated Contracting States: **AT BE CH DE DK ES FR GB GR IT LI LU NL SE**

(71) Applicant: **Matsuda, Ichiro**
**1-16-1-35, Toroku**
**Kumamoto-schi, Kumamoto(JP)**

Applicant: **Nobukuni, Yoshitaka**
**1-10-22, Kotohira**
**Kumamoto-schi, Kumamoto(JP)**

(72) Inventor: **Matsuda, Ichiro**
**1-16-1-35, Toroku**
**Kumamoto-shi, Kumamoto(JP)**
Inventor: **Nobukuni, Yoshitaka**
**1-10-22, Kotohira**
**Kumamoto-shi, Kumamoto(JP)**
Inventor: **Mitsubuchi, Hiroshi**
**1-21-13-401, Suizenji**
**Kumamoto-shi, Kumamoto(JP)**
Inventor: **Asaka, Junichiro**
**1-1-29, Kitahatago-cho-nishi**
**Sakai-shi, Osaka(JP)**
Inventor: **Yaoi, Takeshi**
**2-6-25, Ueno-nishi**
**Toyonaka-shi, Osaka(JP)**

(74) Representative: **Vossius & Partner**
**Siebertstrasse 4 P.O. Box 86 07 67**
**W-8000 München 80(DE)**

## (54) DNA sequence encoding the E1beta subunit of human branched-chain alpha-ketoacid dehydrogenase and detection of mutation thereof.

(57) Provided are a novel nucleotide sequence coding for $E_1\beta$ subunit of human branched-chain $\alpha$-ketoacid dehydrogenase shown in SEQ ID NO: 1 and a method for the detection of the mutation of the gene encoding the human BCKDH-$E_1\beta$, which comprises amplifying a part of the gene, preferably the DNA sequence between position 401 and position 684 in SEQ ID NO: 1, by PCR with the use of all or part of the DNA of the present invention as a probe or a primer and carrying out electrophoresis, preferably non-denatured acrylamide gel electrophoresis, on the PCR products.

According to this method, a DNA diagnosis of MSUD can be carried out.

EP 0 455 037 A2

The present invention relates to a DNA sequence encoding the $E_1\beta$ subunit of human branched-chain $\alpha$-ketoacid dehydrogenase and a method detecting a gene encoding $E_1\beta$ subunit of human branched-chain $\alpha$-ketoacid dehydrogenase according to polymerase chain reaction - mediated single strand conformation polymorphism (PCR-SSCP).

Mammalian branched chain $\alpha$-ketoacid dehydrogenase (BCKDH) [EC 1, 2, 4, 4] is a mitochondrial multienzyme complex catalyzing the oxidative decarboxylation of branched chain $\alpha$-ketoacids derived from branched-chain amino acids such as valine, leucine, and isoleucine, as follows:

$$\text{R-COCOOH} + \text{CoA-SH} + \text{NAD}^+ \xrightarrow{\text{TPP, Mg}^{2+}} \text{R-CO}\sim\text{S-CoA} + CO_2 + \text{NADH} + H^+$$

The BCKDH complex consists of three catalytic components, branched chain $\alpha$-ketoacid decarboxylase ($E_1$), dihydrolipoyl transacylase ($E_2$) and dihydrolipoamide dehydrogenase ($E_3$) components. $E_1$ is further composed of two subunits, $E_1\alpha$ and $E_1\beta$ (Pettit, F. H. et al., Proc. Natl. Acad. Sci. USA. 75:4881-4885, 1978). $E_1$ and $E_2$ components are specific to BCKDH. On the other hand, the $E_3$ component is common among the three ketoacid dehydrogenase complexes, BCKDH, pyruvate dehydrogenase (PDH) and $\alpha$-ketoglutarate dehydrogenase (KGDH) complexes (Yeaman, S. J., Biochem. J. 257:625-632, 1989). The BCKDH complex also contains two specific regulatory enzymes, a kinase (Odessey, R., Biochem. J. 204:353-356, 1982) and a phosphatase (Fatania, H. R. et al., FEBS Lett. 158:234-238, 1983), responsible for regulation of the catalytic activity through phosphorylation and dephosphorylation.

$E_1\alpha$ is the catalytic subunit phosphorylated at two serine residues responsible for regulation of the catalytic activity by covalent modification(Cook, K. G. et al., Eur. J. Biochem. 145:587-591, 1984). The function of $E_1\beta$ is unknown (Yeaman, S. J., Biochem. J. 257:625-632, 1989). $E_2$ catalyzes the transfer of the acyl group from the lipoyl moiety to coenzyme A and forms the structural core of the enzyme complex. To this $E_1$, $E_3$, kinase and phosphatase are bound through noncovalent interactions (Pettit, F. H. et al., Proc. Natl. Acad. Sci. USA. 75:4881-4885, 1978).

Lack of BCKDH activity leads to maple syrup urine disease (MSUD), an autosomal recessive inborn error of metabolism. Several different phenotypes of MSUD have been elucidated on the basis of clinical features, as follows; classical, intermittent, intermediate, thiamine responsive type, $E_2$ deficiency and $E_3$ deficiency. Etiology of MSUD is heterogeneous,as mutations in different regions of any of the BCKDH proteins could lead to decreased functions of the entire complex.

The present inventors reported that a defect in the BCKDH-$E_1\beta$ subunit is one cause of MSUD (Indo, Y. et al., J. Clin. Invest. 80:63-70, 1987). The isolation and characterization of cDNAs encoding all or a part of the human BCKDH-$E_1\alpha$ (Zhang, B. et al., Gene 69:159-164, 1988), BCKDH-$E_2$ component (Hummel, K. B. et al., J. Biol. Chem. 263:6165-6168, 1988) and $E_3$ component (Otulakowski, G. et al., J. Biol. Chem. 262:17313-17318, 1987) have been reported but the primary structure of human BCKDH-$E_1\beta$ has not and the function of this subunit is not well understood. Molecular cloning of BCKDH-$E_1\beta$ had not been successful, despite repeated attempts. Most recently, the present inventors isolated a cDNA clone corresponding to bovine BCKDH-$E_1\beta$ (Nobukuni, Y. et al., Biochemistry 29:1154-1160, 1990).

Lack of human branched chain $\alpha$-ketoacid dehydrogenase (BCKDH) activity leads to maple syrup urine disease (MSUD) which is an autosomal recessive inborn error of metabolism, whereas a defect in the BCKDH-$E_1\beta$ subunit also is one cause of this disease. Accordingly, the isolation of a cDNA for the $E_1\beta$ subunit, the determination of its primary structure and the detection of the mutation of the gene encoding BCKDH-$E_1\beta$ will provide a key to the elucidation of MSUD.

The nucleotide sequence coding for human BCKDH-$E_1\beta$ and deduced amino acid sequence provided by the present invention are shown in SEQ ID NO: 1.

It can be said that the cDNA clone of human BCKDH-$E_1\beta$ is quite useful for examining the structural and functional relationships of the BCKDH complex and for elucidating the molecular mechanisms of MSUD.

Also provided is a method for the detection of the mutation of the gene encoding the $E_1\beta$ subunit of human branched-chain $\alpha$-ketoacid dehydrogenase, which comprises amplifying a part of the gene, preferably the DNA sequence between position 401 and position 684 in SEQ ID NO: 1, by PCR with the use of all or part of the DNA of the present invention as a probe or a primer and carrying out electrophoresis, preferably non-denatured acrylamide gel electrophoresis, on the PCR products.

According to this method, a DNA diagnosis of MSUD can be carried out.

Figure 1 shows a restriction map and sequencing strategy for human BCKDH-$E_1\beta$ cDNA.

The present inventors suspected that the nucleotide sequence and the primary structure of human BCKDH-$E_1\beta$ has to be determined to analyze the alterations seen in studies on MSUD patients, and carried out cDNA cloning of the human BCKDH-$E_1\beta$ in an attempt to elucidate the molecular basis of the disease.

First of all, the present inventors isolated and characterized a 1.35 kbp cDNA clone encoding the entire precursor of the BCKDH $E_1\beta$ subunit from a human placental cDNA library. Nucleotide sequence analysis revealed that the isolated cDNA clone (λ hBE$_1\beta$ -1) contained a 5'-untranslated sequence of 36 nucleotides, the translated sequence of 1,176 nucleotides and the 3'-untranslated sequence of 169 nucleotides. Comparison of the amino acid sequence predicted from the nucleotide sequence of the cDNA insert of the clone with the N-terminal amino acid sequence of the purified mature bovine BCKDH-$E_1\beta$ subunit showed that the cDNA insert encodes for a 342 amino acid subunit with a molecular weight of 37,585. The subunit is synthesized as the precursor with a leader sequence of 50 amino acids and is processed at the N-terminus. A search for protein homology revealed that the primary structure of human BCKDH-$E_1\beta$ was similar to the bovine BCKDH-$E_1\beta$ and to the $E_1\beta$ subunit of human pyruvate dehydrogenase complex, in all regions. The structures and functions of mammalian α-ketoacid dehydrogenase complexes are apparently highly conserved.

The nucleotide sequence coding for human BCKDH-$E_1\beta$ and deduced amino acid sequence according to the present invention are shown in SEQ ID NO: 1. This nucleotide sequence, its degenerated sequences and allelic sequences thereof can be used for the production of BCKDH-$E_1\beta$ by recombinant DNA processes. Thus, the present invention also relates to recombinant vectors and expression vectors containing said nucleotide sequences. The present invention further relates to a process for the production of BCKDH-$E_1\beta$ comprising the steps of culturing a recombinant host organism, preferably a bacterium, a yeast or another fungus, or a mammalian cell transformed with such a recombinant vector under suitable conditions and recovering the expression product BCKDH-$E_1\beta$ from the culture medium. Accordingly the present invention also relates to BCKDH-$E_1\beta$ polypeptides encoded by the DNA sequences of the present invention.

Moreover, the present inventors established a method for the detection of the mutation of the gene encoding the $E_1\beta$ subunit of human branched-chain α-ketoacid dehydrogenase, which comprises amplifying a part of the gene, preferably the DNA sequence between position 401 and position 684 in SEQ ID NO: 1, by PCR with the use of all or part of the DNA of the present invention as a probe or a primer and carrying out electrophoresis, preferably non-denatured acrylamide gel electrophoresis, on the PCR products.

Primers of 15 to 40 bases containing at least continuous 15 bases of the DNA sequences shown in SEQ ID NOs: 2 to 13 is used for the PCR.

The DNA sequence shown in SEQ ID NO: 2 is a sense sequence corresponding to 1 to 26 of the DNA sequence shown in SEQ ID NO: 1, the DNA sequence shown in SEQ ID NO: 3 is an anti-sense sequence corresponding to 253 to 276 of the DNA sequence shown in SEQ ID NO: 1, the DNA sequence shown in SEQ ID NO: 4 is a sense sequence corresponding to 183 to 206 of the DNA sequence shown in SEQ ID NO: 1, the DNA sequence shown in SEQ ID NO: 5 is an anti-sense sequence corresponding to 426 to 449 of the DNA sequence shown in SEQ ID NO: 1, the DNA sequence shown in SEQ ID NO: 6 is a sense sequence corresponding to 401 to 424 of the DNA sequence shown in SEQ ID NO: 1, the DNA sequence shown in SEQ ID NO: 7 is an anti-sense sequence corresponding to 661 to 684 of the DNA sequence shown in SEQ ID NO: 1, the DNA sequence shown in SEQ ID NO: 8 is a sense sequence corresponding to 634 to 657 of the DNA sequence shown in SEQ ID NO: 1, the DNA sequence shown in SEQ ID NO: 9 is an anti-sense sequence corresponding to 882 to 905 of the DNA sequence shown in SEQ ID NO: 1, the DNA sequence shown in SEQ ID NO: 10 is a sense sequence corresponding to 846 to 869 of the DNA sequence shown in SEQ ID NO: 1, the DNA sequence shown in SEQ ID NO: 11 is an anti-sense sequence corresponding to 1118 to 1141 of the DNA sequence shown in SEQ ID NO: 1, the DNA sequence shown in SEQ ID NO: 12 is a sense sequence corresponding to 1042 to 1065 of the DNA sequence shown in SEQ ID NO: 1, and the DNA sequence shown in SEQ ID NO: 13 is an anti-sense sequence corresponding to 1280 to 1305 of the DNA sequence shown in SEQ ID NO: 1.

Each pair of a sense primer and an anti-sense primer and the DNA fragments amplified thereby are shown in Table 1.

Table 1

| Region (base No.) | Sense primer | Antisense primer |
|---|---|---|
| I (1 - 276) | SEQ ID NO: 2 | SEQ ID NO: 3 |
| II (183 - 449) | SEQ ID NO: 4 | SEQ ID NO: 5 |
| III (401 - 684) | SEQ ID NO: 6 | SEQ ID NO: 7 |
| IV (634 - 905) | SEQ ID NO: 8 | SEQ ID NO: 9 |
| V (846 - 1141) | SEQ ID NO: 10 | SEQ ID NO: 11 |
| VI (1042 - 1305) | SEQ ID NO: 12 | SEQ ID NO: 13 |

Preferably PCR carried out in this invention is a reverse transcription - polymerase chain reaction (RT-PCR), where the reverse transcription is carried out before the PCR. As an RT-primer used in the reverse transcription, a primer of 15 to 40 bases containing at least continuous 15 bases of the DNA sequence shown in SEQ ID NO: 14 may be used. The DNA sequence shown in SEQ ID NO:14 is an anti-sense sequence corresponding to 1307 to 1332 of the DNA sequence shown in SEQ ID NO:1.

As the primer used in the method for detecting the mutation of the gene encoding human BCKDH-$E_1\beta$ according to this invention, not only the DNA sequence shown in SEQ ID NOs: 2 to 14 but also the DNA sequence of 15 to 40 bases containing at least continuous 15 bases thereof can be used. 15 to 40 bases is a normal length as a primer used in PCR. Moreover, pairs of primers complementary to the DNA sequence shown in SEQ ID NOs: 2 to 14 can also be used. A design and preparation of primers may be performed in accordance with the DNA sequence shown in SEQ ID NO: 1, so that the gene encoding human BCKDH-$E_1\beta$ can be specifically detected by PCR.

The PCR products are detected by diluting with formamide pigment solution, electrophoresing, and exposing on X-ray film. Complementary strands of double stranded DNA is separated from each other by carrying out non-denatured acrylamide gel electrophoresis. A mobility of each complementary strand depends on its secondary or higher structure which is specific for the nucleotide sequence thereof. Therefore, a normal DNA fragment and a mutated DNA fragment each is detected according to the difference of the mobility between them. Single strand conformation polymorphism (SSCP) is a method for rapidly detecting the mutation of the patient's gene by using this principle (Hayashi, K. et al., Jikken Igaku, vol. 8, No. 9, 1133 - 1136, 1991).

With sequencing the DNA fragment whose mobility is different from that of normal one, more precise detection of the mutation may be performed.

The present invention will be further illustrated by reference to the following example, but it is not intended to restrict the present invention.

## Example

### 1. Determination of the DNA sequence encoding human BCKDH-$E_1\beta$

Restriction enzymes, pUC18 vector DNAs, 7-DEAZA Sequencing Kits and random primer labelling kits were purchased from Takara Shuzo Co. (Kyoto, Japan). Nitrocellulose hybridization membranes were from Schleicher and Schuell (Dassel, West Germany). [$\alpha$-$^{32}$P] dCTP (specific activity, 3,000 Ci/mmol) were from Amersham (Arlington Heights, IL).

### Isolation of cDNA

The 1.7 kbp cDNA clone (λ $bE_1\beta$ -2) for bovine BCKDH-$E_1\beta$ was isolated using the mixture of synthetic oligonucleotides (17 mer, 24 mixture) as a probe for a bovine liver cDNA library constructed in λ gtll (Nobukuni, Y. et al., Biochemistry 29:1154-1160, 1990). Approximately $6 \times 10^5$ recombinant phage plaques were screened from the human placental cDNA library constructed in λ gtll (Ye, R. D. et al., J. Biol. Chem. 262:3718-3725, 1987) using the 1.7 kbp cDNA clone (λ $bE_1\beta$ -2) as a probe. The insert was labeled with [$\alpha$-$^{32}P$] dCTP (3,000 Ci/mmol) using the random primer labelling kit. Prehybridization, hybridization and washing of nitrocellulose filters were as described by Maniatis, T. et al., Molecular Cloning, 1982. Hybridizing plaques detected by autoradiography were picked up from the mother agar plate. Successive screenings were carried out, using fewer and fewer plaques at each step until well-isolated phage plaques had been cloned.

Restriction endonuclease map and nucleotide sequence

Recombinant phage DNA was prepared as described in Molecular Cloning (supra). EcoRI-excised cDNA inserts were subcloned into plasmid vector pUC18 and characterized by restriction endonuclease mapping. Restriction fragments were subcloned into pUC18 for sequencing. In addition, ordered serial deletions from the 5'→3' end of both strands of the pUC18 insert were produced with exonuclease III/mung bean nuclease for sequencing (Henikoff, S., Gene 28:351-359, 1984). DNA sequencing was performed by the dideoxy chain termination method (Sanger, F. et al., Proc. Natl. Acad. Sci. USA 74:5463-5467, 1977) using an alkali-denatured plasmid as the template (Hattori, M. et al., Anal. Biochem. 152:232-238, 1986).

Protein data base search

Homologous amino acid sequences were sought in the GenBank (Release 59.0)/EMBL (Release 18.0) protein data base on a VAX computer using the WORDSEARCH program (Version 6.0, April 1989). The SEGMENTS program was used for the alignment procedure.

2. Detection of a mutation of a gene encoding human BCKDH-$E_1\beta$ by PCR-SSPC

Reverse transcriptase and reverse transcriptase reaction solution were purchased from BPL. Taq polymerase and taq polymerase reaction solution for PCR were from Takara Shuzo Co.. Primers were prepared with an automatic DNA synthesizer (ABI). [$\gamma$ -$^{32}P$] ATP (specific activity, 5,000 Ci/mmole) was from Amersham. Amplification of DNA by PCR was performed by using Cirmal Cycler (Cetus).

Preparation of first strand cDNA

The first strand cDNA was synthesized by reverse transcription with the use of total RNA (30 $\mu$g) derived from MSUD patients as a template and RT-primer shown in SEQ ID NO: 14.

Amplification of DNA in specific regions of $E_1\beta$ by PCR

PCR primer having the DNA sequence shown in SEQ ID NOs: 3 to 14 were labelled with [$\gamma$ -$^{32}P$] ATP (5,000 Ci/mmole) in accordance with the method of Maniatis, T. et al., Molecular Cloning, 2nd edition, 1989. With the use of pairs of these primers shown in Table 2 and the first strand cDNA synthesized above as a template, six DNA fragments (regions I to VI, see Table 2) present in the DNA sequence of SEQ ID NO: 1 were amplified by PCR.

Table 2

| Region (base No.) | Sense primer | Antisense primer |
|---|---|---|
| I (1 - 276) | SEQ ID NO: 2 | SEQ ID NO: 3 |
| II (183 - 449) | SEQ ID NO: 4 | SEQ ID NO: 5 |
| III (401 - 684) | SEQ ID NO: 6 | SEQ ID NO: 7 |
| IV (634 - 905) | SEQ ID NO: 8 | SEQ ID NO: 9 |
| V (846 - 1141) | SEQ ID NO: 10 | SEQ ID NO: 11 |
| VI (1042 - 1305) | SEQ ID NO: 12 | SEQ ID NO: 13 |

Detection of a mutation in the regions amplified

The DNA solution having the nucleotides amplified by PCR were diluted with formamide pigment solution and electrophoresed on 6% non-denatured acryl amide gel (height 40cm) at 4°C and 40W for 3 hours. After electrophoresis, the gel was dried and exposed on X-ray film. Detailed manner was in accordance with the methods of Orita, M. et al., Proc. Natl. Acad. Sci. USA, 86: 2766 - 2770, 1989; Orita, M. et al., Genomics, 5: 874 - 879, 1989; Hayashi, K. et al., Nucleic Acids Res., 17: 3605, 1989; and Hayashi, K. et al., Jikken Igaku 8(9): 1133-1136, 1990.

Results

To isolate the human BCKDH-$E_1\beta$ cDNA clone, the present inventors initially screened approximately 1 $\times 10^6$ recombinant phage plaques of a human placental cDNA library constructed in λ gtll, with a specific rabbit antibody raised against bovine BCKDH-$E_1\beta$, and obtained five positive clones. All were found to be false positive by nucleotide sequencing.

The present inventors then isolated and characterized a cDNA clone coding for bovine BCKDH-$E_1\beta$ from a bovine liver cDNA library constructed in λ gtll by screening with a mixture of synthetic oligonucleotide probes corresponding to the C-terminal 5-residue sequence of the bovine BCKDH-$E_1\beta$ - (Nobukuni, Y. et al., supra). Using this 1.7 kbp EcoRI fragment of bovine BCKDH-$E_1\beta$ cDNA insert (λ b$E_1\beta$ -2) as a probe, approximately 6 $\times 10^6$ plaque-forming units were screened from a human placental cDNA library. Only one positive clone ( λ h$E_1\beta$ -1) was plaque-purified to homogeneity through five successive rounds of screening, and the cDNA inserts were subcloned into the EcoRI site of pUC18 for further characterization. This recombinant phage clone insert had 1.35 kbp.

The restriction endonuclease map of a cDNA insert from the phage clone λ hB$E_1\beta$ -1 and sequencing strategy for the insert are shown in Fig. 1. The nucleotide sequence and deduced amino acid sequence are shown in SEQ ID NO: 1. The λ hB$E_1\beta$ -1 insert is composed of 1,381 bp consisting of a 36 bp 5'-untranslated sequence, a 1,176 bp long open reading frame, and 169 bp 3'-untranslated sequence. The nucleotide sequence surrounding the putative initiation codon, GGGGAUGG, is rather different from the consensus sequence of CC$^{A}_{G}$ CCAUGG (Kozak, L. M. et al., Nucl. Acids. Res. 12:857-872, 1984). A polyadenylation signal of the type AATAAT (Birnstiel, M. L. et al., Cell 41:349-359, 1985) is found 7 bp upstream of a poly(A) tail.

The open reading frame could be translated into a 392 amino acid residue protein. Comparison of the amino acid sequence predicted from the nucleotide sequence of the clone cDNA insert with the N-terminal amino acid sequence of purified bovine BCKDH-$E_1\beta$ determined by Edman degradation (Nobukuni, Y. et al., Biochemistry 29:1154-1160, 1990) revealed that the N-terminal 50 amino acid residues of the putative

precursor protein are missing the mature $E_1\beta$. BCKDH-$E_1\beta$ is a nuclear encoded mitochondrial protein, the precursor of which contains a leader sequence of 50 amino acid residues. Comparison of the putative $E_1\beta$ leader sequence (negatively numbered amino acid residues in SEQ ID NO: 1) with those of other mitochondrial proteins revealed a number of common features. The putative $E_1\beta$ leader sequence contains periodically spaced basic amino acids rich in Leu and Arg and has few acidic residues (only one Asp as residue -7). These findings are compatible with those proposed for the leader sequence of mitochondrial targetting enzymes (von Heijne, G., EMBQ J. 5:1355-1342, 1986). On the basis of these findings, the molecular mass of the $E_1\beta$ precursor is estimated to be 43,130 and that of the mature $E_1\beta$ is 37,585, in good agreement with the 37,000 estimated by immunoblot analysis (Indo, Y. et al., J. Clin. Invest. 80:63-70, 1987).

A protein homology search revealed that the primary structure of human BCKDH-$E_1\beta$ is similar to the human PDH-$E_1\beta$ (Koike, K. et al., Proc. Natl. Acad. Sci. USA 85:41-45, 1988), in all regions. 98% of the amino acid residues of human BCKDH-$E_1\beta$ are identical to bovine BCKDH-$E_1\beta$ and 33% of the amino acid residues of human BCKDH-$E_1\beta$ are identical to the corresponding residues of PDH-$E_1\beta$; this similarity increased to 82% if conservative amino-acid substitutions are taken into account. It has been demonstrated that mammalian $\alpha$-ketoacid dehydrogenase complexes such as PDH, BCKDH, and KGDH are functionally and structurally similar. The amino acid sequence of the mammalian BCKDH-$E_1\alpha$ is highly homologous to that of mammalian PDH-$E_1\alpha$ (Zhang, B, et al., Gene 69:159-164, 1988); the same is true of BCKDH-$E_2$ - (Hummel, K. B. et al., J. Biol. Chem. 263:6165-6168, 1988). The present invention showed that human BCKDH-$E_1\beta$ is also similarly homologous to human PDH-$E_1\beta$. Extensive homologies between BCKDH-$E_1\beta$ and PHD-$E_1\beta$ throughout the primary structure suggest that the secondary structure, tertiary structure and function are also similar. Furthermore BCKDH-$E_1\beta$ and PDH-$E_1\beta$ may possibly arise from a common ancestral gene, although the function of $E_1\beta$ has yet to be determined. A highly conserved structure of the $E_1\beta$-subunit of 2-oxo acid dehydrogenases of mammals suggests that the $E_1\beta$ -subunit of these enzymes no doubt plays important roles in enzyme activities.

A defect in the BCKDH-complex results in MSUD. The etiology of this disease is heterogenous as mutations in different regions of any of the BCKDH proteins could lead to decreased functions of the entire complex. Recently, a defect of $E_2$, a defect of $E_1\beta$, mutation of $E_1\alpha$ and loss of $E_1\alpha$ and $E_1\beta$ subunits have been detected in MSUD patients.

The present inventors analyzed the biochemical basis for clinical heterogeneity in MSUD and noted a defect in $E_1\beta$ as one cause of the disease. In these cases, immunologically cross reactive material corresponding to BCKDH-$E_1\beta$ was not detected. $E_1\beta$ is associated with $E_1\alpha$ by non-covalent binding and the $E_1$ subunit is attached to $E_2$. Thus, the absence of $E_1\beta$ theoretically could result from abnormalities in structures of $E_1\alpha$ or $E_2$. The absence of $E_1\beta$ might not indicate a genetic abnormality in the $E_1\beta$ gene. A detailed analysis of $E_1\beta$ deficiency suggested that the mutations might be heterogenous. The $E_1\beta$ deficiency will have to be analyzed at the gene level.

To elucidate the molecular mechanism of $E_1\beta$ deficiency, the present inventors first analyzed the expression of $E_1\beta$ transcript in lymphoblastoid cell lines in which $E_1\beta$ was not detected by immunoblot analysis. The $E_1\beta$ transcript was not clearly defined by northern blot analysis using poly(A)$^+$ RNA (10-15 $\mu$g), even in normal control lymphoblastoid cell lines. The quantity of the $E_1\beta$ transcript may be low or the transcript may readily degrade, a proposal based on the finding that the present inventors could obtain only one clone when screening the cDNA-library using the nucleotide probe ($\lambda$ b$E_1\beta$ -2), as described above.

Moreover, the present inventors attempted to detect the mutation of the gene encoding $E_1\beta$ with PCR-SSCP. As a result, difference in mobility between the DNA fragment of normal human and that of MSUD patients was observed in the region III.

## Claims

1. A DNA sequence encoding the amino acid sequence shcwn in SEQ ID NO: 1.

2. The DNA sequence of claim 1, wherein said amino acid sequence is that of from Met at position -50 to Tyr at position 342 in SEQ ID NO: 1.

3. The DNA sequence of claim 1, wherein said amino acid sequence is that of from Val at position 1 to Tyr at position 342 in SEQ ID NO: 1.

4. The DNA sequence of claim 1, which is the DNA sequence shown in SEQ ID NO:1.

5. A method for detecting a mutation of a gene encoding the $E_1\beta$ subunit of human branched-chain $\alpha$-ketoacid dehydrogenase, which comprises amplifying a part of the gene by PCR and carrying out electrophoresis on the PCR products.

6. The method of claim 5, wherein the electrophoresis is non-denatured acrylamide gel electrophoresis.

7. The method of claim 5 or 6, wherein the part of the gene is a mutated part.

8. The method of any one of claims 5 to 7, wherein the part of the gene is between position 401 and position 684 in SEQ ID NO: 1.

9. The method of any one of claims 5 to 8, which is based on the difference of mobility between a normal DNA fragment and a mutated DNA fragment of the gene.

10. The method according to any one of claims 5 to 9 wherein at least one primer according to any one of SEQ ID NO: 2 to SEQ ID NO: 13 is used.

11. A kit for detecting a mutation of a gene encoding the $E_1\beta$ subunit of human branched-chain $\alpha$-ketoacid dehydrogenase, containing at least a part of a DNA sequence according to SEQ ID NO: 1 or at least one primer according to any one of SEQ ID NO: 2 to 13.

**Claims for the following Contracting State: ES**

1. A method for the production of a DNA sequence encoding the amino acid sequence shown in SEQ ID NO: 1 comprising the screening of a human cDNA library with a BCKDH-$E_1\beta$ probe.

2. The method of claim 1, wherein said amino acid sequence is that of from Met at position -50 to Tyr at position 342 in SEQ ID NO: 1.

3. The method of claim 1, wherein said amino acid sequence is that of from Val at position 1 to Tyr at position 342 in SEQ ID NO: 1.

4. The method of claim 1, wherein said DNA sequence is the DNA sequence shown in SEQ ID NO: 1.

5. A method for detecting a mutation of a gene encoding the $E_1\beta$ subunit of human branched-chain $\alpha$-ketoacid dehydrogenase, which comprises amplifying a part of the gene by PCR and carrying out electrophoresis on the PCR products.

6. The method of claim 5, wherein the electrophoresis is non-denatured acrylamide gel electrophoresis.

7. The method of claim 5 or 6, wherein the part of the gene is a mutated part.

8. The method of any one of claims 5 to 7, wherein the part of the gene is between position 401 and position 684 in SEQ ID NO: 1.

9. The method of any one of claims 5 to 8, which is based on the difference of mobility between a normal DNA fragment and a mutated DNA fragment of the gene.

10. The method according to any one of claims 5 to 9 wherein at least one primer according to any one of SEQ ID NO: 2 to SEQ ID NO: 13 is used.

Fig. 1

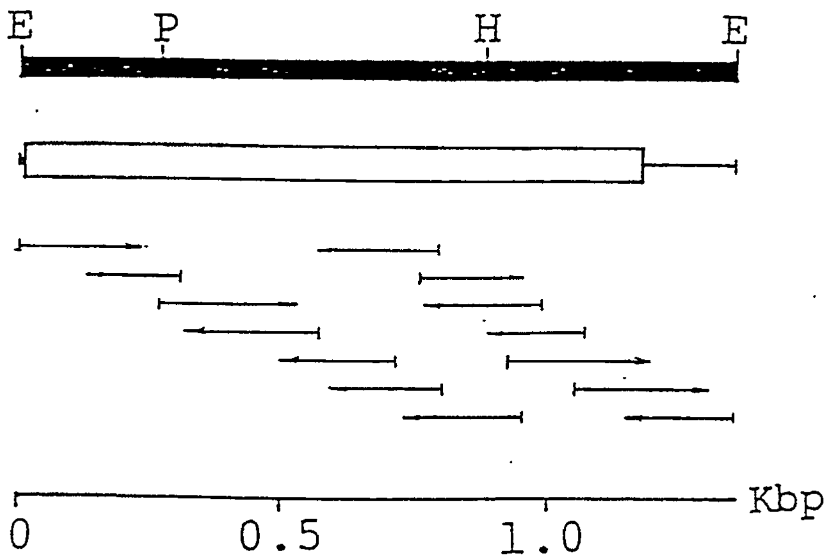


■ : $\lambda hBE_1\beta$ - 1 insert

□ : coding region

E : EcoRI

P : PstI

H : HindIII

SEQUENCE LISTING

INFORMATION FOR SEQ ID NO: 1

(i) SEQUENCE CHARACTERISTICS:

(A) LENGTH: 1381 base pairs

(B) TYPE: nucleic acid

(C) STRANDEDNESS: single

(D) TOPOLOGY: linear

(ii) MOLECULE TYPE: cDNA to mRNA

(xi) SEQUENCE DESCRIPTION: SEQ ID NO: 1 :

```
CGGCTGCAGA GCCTGAGAAT CCCGGTGTGA GCGGGG ATG GCG GTT GTA GCG GCG      54
                                        Met Ala Val Val Ala Ala
                                        -50                 -45
GCT GCC GGC TGG CTA CTC AGG CTC AGG GCG GCA GGG GCT GAG GGG CAC     102
Ala Ala Gly Trp Leu Leu Arg Leu Arg Ala Ala Gly Ala Glu Gly His
                -40                 -35                 -30
TGG CGT CGG CTT CCT GGC GCG GGG CTG GCG CGG GGC TTT TTG CAC CCC     150
Trp Arg Arg Leu Pro Gly Ala Gly Leu Ala Arg Gly Phe Leu His Pro
            -25                 -20                 -15
GCC GCG ACT GTC GAG GAT GCG GCC CAG AGG CGG CAG GTG GCT CAT TTT     198
Ala Ala Thr Val Glu Asp Ala Ala Gln Arg Arg Gln Val Ala His Phe
        -10                 -5                  1
ACT TTC CAG CCA GAT CCG GAG CCC CGG GAG TAC GGG CAA ACT CAG AAA     246
Thr Phe Gln Pro Asp Pro Glu Pro Arg Glu Tyr Gly Gln Thr Gln Lys
    5                   10                  15                  20
```

ATG AAT CTT TTC CAG TCT GTA ACA AGT GCC TTG GAT AAC TCA TTG GCC 294
Met Asn Leu Phe Gln Ser Val Thr Ser Ala Leu Asp Asn Ser Leu Ala
25 30 35
AAA GAT CCT ACT GCA GTA ATA TTT GGT GAA GAT GTT GCC TTT GGT GGA 342
Lys Asp Pro Thr Ala Val Ile Phe Gly Glu Asp Val Ala Phe Gly Gly
40 45 50
GTC TTT AGA TGC ACT GTT GGC TTG CGA GAC AAA TAT GGA AAA GAT AGA 390
Val Phe Arg Cys Thr Val Gly Leu Arg Asp Lys Tyr Gly Lys Asp Arg
55 60 65
GTT TTT AAT ACC CCA TTG TGT GAA CAA GGA ATT GTT GGA TTT GGA ATC 438
Val Phe Asn Thr Pro Leu Cys Glu Gln Gly Ile Val Gly Phe Gly Ile
70 75 80
GGA ATT GCG GTC ACT GGA GCT ACT GCC ATT GCG GAA ATT CAG TTT GCA 486
Gly Ile Ala Val Thr Gly Ala Thr Ala Ile Ala Glu Ile Gln Phe Ala
85 90 95 100
GAT TAT ATT TTC CCT GCA TTT GAT CAG ATT GTT AAT GAA GCT GCC AAG 534
Asp Tyr Ile Phe Pro Ala Phe Asp Gln Ile Val Asn Glu Ala Ala Lys
105 110 115
TAT CGC TAT CGC TCT GGG GAT CTT TTT AAC TGT GGA AGC CTC ACT ATC 582
Tyr Arg Tyr Arg Ser Gly Asp Leu Phe Asn Cys Gly Ser Leu Thr Ile
120 125 130
CGG TCC CCT TGG GGC TGT GTT GGT CAT GGG GCT CTC TAT CAT TCT CAG 630
Arg Ser Pro Trp Gly Cys Val Gly His Gly Ala Leu Tyr His Ser Gln
135 140 145
AGT CCT GAA GCA TTT TTT GCC CAT TGC CCA GGA ATC AAG GTG GTT ATA 678
Ser Pro Glu Ala Phe Phe Ala His Cys Pro Gly Ile Lys Val Val Ile
150 155 160
CCC AGA AGC CCT TTC CAG GCC AAA GGA CTT CTT TTG TCA TGC ATA GAG 726
Pro Arg Ser Pro Phe Gln Ala Lys Gly Leu Leu Leu Ser Cys Ile Glu
165 170 175 180

GAT AAA AAT CCT TGT ATA TTT TTT GAA CCT AAA ATA CTT TAC AGG GCA 774
Asp Lys Asn Pro Cys Ile Phe Phe Glu Pro Lys Ile Leu Tyr Arg Ala
185 190 195

GCA GCG GAA GAA GTC CCT ATA GAA CCA TAC AAC ATC CCA CTG TCC CAG 822
Ala Ala Glu Glu Val Pro Ile Glu Pro Tyr Asn Ile Pro Leu Ser Gln
200 205 210

GCC GAA GTC ATA CAG GAA GGG AGT GAT GTT ACT CTA GTT GCC TGG GGC 870
Ala Gln Val Ile Gln Glu Gly Ser Asp Val Thr Leu Val Ala Trp Gly
215 220 225

ACT CAG GTT CAT GTG ATC CGA GAG GTA GCT TCC ATG GCA AAA GAA AAG 918
Thr Gln Val His Val Ile Arg Glu Val Ala Ser Met Ala Lys Gln Lys
230 235 240

CTT GGA GTG TCT TGT GAA GTC ATT GAT CTG AGG ACT ATA ATA CCT TGG 966
Leu Gly Val Ser Cys Glu Val Ile Asp Leu Arg Thr Ile Ile Pro Trp
245 250 255 260

GAT GTG GAC ACA ATT TGT AAG TCT GTG ATC AAA ACA GGG CGA CTG CTA 1014
Asp Val Asp Thr Ile Cys Lys Ser Val Ile Lys Thr Gly Arg Leu Leu
265 270 275

ATC AGT CAC GAG GCT CCC TTG ACA GGC GGC TTT GCA TCG GAA ATC AGC 1062
Ile Ser His Glu Ala Pro Leu Thr Gly Gly Phe Ala Ser Glu Ile Ser
280 285 290

TCT ACA GTT CAG GAG GAA TGT TTC TTG AAC CTA GAG GCT CCT ATA TCA 1110
Ser Thr Val Gln Glu Glu Cys Phe Leu Asn Leu Glu Ala Pro Ile Ser
295 300 305

AGA GTA TGT GGT TAT GAC ACA CCA TTT CCT CAC ATT TTT GAA CCA TTC 1158
Arg Val Cys Gly Tyr Asp Thr Pro Phe Pro His Ile Phe Glu Pro Phe
310 315 320

TAC ATC CCA GAC AAA TGG AAG TGT TAT GAT GCC CTT CGA AAA ATG ATC 1206
Tyr Ile Pro Asp Lys Trp Lys Cys Tyr Asp Ala Leu Arg Lys Met Ile
325 330 335 340

AAC TAT TGACCATATA GGTAGGTATG CATCTTGAGA AAGCTACTAT GTGCCCCTGA 1262
Asn Tyr

CATTAACGTA CTGTTAACCA AGACACAGCA ATCATCAGTG TTTTGATGGT AACAAACTTT 1322
GATGGTAAAG TTGATAAAAG GCAACTTTCA GAAGAAAATA ATGTGCTTTA AAAAAAAAA 1381

INFORMATION FOR SEQ ID NO: 2

( i ) SEQUENCE CHARACTERISTICS:

(A) LENGTH: 26 base pairs

(B) TYPE: nucleic acid

(C) STRANDEDNESS: single

(D) TOPOLOGY: linear

( iv ) ANTI SENSE: No

(xi) SEQUENCE DESCRIPTION: SEQ ID NO: 2 :

CGGCTGCAGA GCCTGAGAAT CCCGGT 26

INFORMATION FOR SEQ ID NO: 3

( i ) SEQUENCE CHARACTERISTICS:

(A) LENGTH: 24 base pairs

(B) TYPE: nucleic acid

(C) STRANDEDNESS: single

(D) TOPOLOGY: linear

(iv) ANTI SENSE: Yes

(xi) SEQUENCE DESCRIPTION: SEQ ID NO: 3 :

GGCACTTGTT ACAGACTGGA AAAG 24

INFORMATION FOR SEQ ID NO: 4

(i) SEQUENCE CHARACTERISTICS:

(A) LENGTH: 24 base pairs

(B) TYPE: nucleic acid

(C) STRANDEDNESS: single

(D) TOPOLOGY: linear

(iv) ANTI SENSE: No

(xi) SEQUENCE DESCRIPTION: SEQ ID NO: 4 :

GCAGGTGGCT CATTTTACTT TCCA 24

INFORMATION FOR SEQ ID NO: 5

(i) SEQUENCE CHARACTERISTICS:

(A) LENGTH: 24 base pairs

(B) TYPE: nucleic acid

(C) STRANDEDNESS: single

(D) TOPOLOGY: linear

(iv) ANTI SENSE: Yes

(xi) SEQUENCE DESCRIPTION: SEQ ID NO: 5 :

ACCGCAATTC CGATTCCAAA TCCA 24

INFORMATION FOR SEQ ID NO: 6

(i) SEQUENCE CHARACTERISTICS:

(A) LENGTH: 24 base pairs

(B) TYPE: nucleic acid

(C) STRANDEDNESS: single

(D) TOPOLOGY: linear

(iv) ANTI SENSE: No

(xi) SEQUENCE DESCRIPTION: SEQ ID NO: 6 :

CCCCATTGTG TGAACAAGGA ATTG 24

INFORMATION FOR SEQ ID NO: 7

(i) SEQUENCE CHARACTERISTICS:

(A) LENGTH: 24 base pairs

(B) TYPE: nucleic acid

(C) STRANDEDNESS: single

(D) TOPOLOGY: linear

(iv) ANTI SENSE: Yes

(xi) SEQUENCE DESCRIPTION: SEQ ID NO: 7 :

TCTGGGTATA ACCACCTTGA TTCC 24

INFORMATION FOR SEQ ID NO: 8

(i) SEQUENCE CHARACTERISTICS:

(A) LENGTH: 24 base pairs

(B) TYPE: nucleic acid

(C) STRANDEDNESS: single

(D) TOPOLOGY: linear

(iv) ANTI SENSE: No

(xi) SEQUENCE DESCRIPTION: SEQ ID NO: 8 :

CCTGAAGCAT TTTTTGCCCA TTGC 24

INFORMATION FOR SEQ ID NO: 9

(i) SEQUENCE CHARACTERISTICS:

(A) LENGTH: 24 base pairs

(B) TYPE: nucleic acid

(C) STRANDEDNESS: single

(D) TOPOLOGY: linear

(iv) ANTI SENSE: Yes

(xi) SEQUENCE DESCRIPTION: SEQ ID NO: 9 :

ATGGAAGCTA CCTCTCGGAT CACA 24

INFORMATION FOR SEQ ID NO: 10

(i) SEQUENCE CHARACTERISTICS:

(A) LENGTH: 24 base pairs

(B) TYPE: nucleic acid

(C) STRANDEDNESS: single

(D) TOPOLOGY: linear

(iv) ANTI SENSE: No

(xi) SEQUENCE DESCRIPTION: SEQ ID NO: 10 :

TGATGTTACT CTAGTTGCCT GGGG 24

INFORMATION FOR SEQ ID NO: 11

(i) SEQUENCE CHARACTERISTICS:

(A) LENGTH: 24 base pairs

(B) TYPE: nucleic acid

(C) STRANDEDNESS: single

(D) TOPOLOGY: linear

( iv ) ANTI SENSE: Yes

(xi) SEQUENCE DESCRIPTION: SEQ ID NO: 11 :

GAGGAAATGG TGTGTCATAA CCAC 24

INFORMATION FOR SEQ ID NO: 12

( i ) SEQUENCE CHARACTERISTICS:

(A) LENGTH: 24 base pairs

(B) TYPE: nucleic acid

(C) STRANDEDNESS: single

(D) TOPOLOGY: linear

( iv ) ANTI SENSE: No

(xi) SEQUENCE DESCRIPTION: SEQ ID NO: 12 :

GGCTTTGCAT CGGAAATCAG CTCT 24

INFORMATION FOR SEQ ID NO: 13

( i ) SEQUENCE CHARACTERISTICS:

(A) LENGTH: 26 base pairs

(B) TYPE: nucleic acid

(C) STRANDEDNESS: single

(D) TOPOLOGY: linear

( iv ) ANTI SENSE: Yes

(xi) SEQUENCE DESCRIPTION: SEQ ID NO: 13 :

AAACACTGAT GATTGCTGTG TCTTGG 26

INFORMATION FOR SEQ ID NO: 14

( i ) SEQUENCE CHARACTERISTICS:

(A) LENGTH: 26 base pairs

(B) TYPE: nucleic acid

(C) STRANDEDNESS: single

(D) TOPOLOGY: linear

( iv ) ANTI SENSE: Yes

(xi) SEQUENCE DESCRIPTION: SEQ ID NO: 14 :

CTTTACCATC AAAGTTTGTT ACCATC 26